# EUROPEAN PATENT SPECIFICATION

(11) **EP 0 626 160 B1**
(45) Date of publication and mention of the grant of the patent: **21.01.1998**
(21) Application number: 94108161.4
(22) Date of filing: 26.05.1994
(51) Int. Cl.: A61F 13/15

(54) **Absorbent article**
Absorbierendes Produkt
Article absorbant

(30) Priority: 26.05.1993 JP 145361/93
(43) Date of publication of application: 30.11.1994
(73) Proprietor: JAPAN ABSORBENT TECHNOLOGY INSTITUTE, Chuo-ku, Tokyo (JP)
(72) Inventor: Suzuki, Migaku, Kamakura-shi, Kanagawa (JP); Fukui, Hiroaki, Kawaguchi-shi, Saitama (JP)
(74) Representative: Klingseisen, Franz, Dipl.-Ing.

(56) References cited:
- EP-A- 0 357 298
- EP-A- 0 412 549
- EP-A- 0 446 867
- EP-A- 0 510 715
- EP-A- 0 522 584
- EP-A- 0 563 971
- EP-A- 0 568 085
- WO-A-93/25170

## Description

The invention relates to an absorbent article according to the preamble of claim 1.

Such an absorbent article is known from EP-A-0 357 298 which discloses a disposable absorbent article, such as a diaper having a liquid impervious backsheet, an urine pervious liner and an absorbent core intermediate the backsheet and the liner. The liner has a passageway to allow communication of solid waste materials to the core, thereby isolating such waste from the skin of the wearer. The liner is longitudinally contracted by elastic strands disposed longitudinally nonadjacent the passageway, improving the fit of the article to the wearer without sacrificing leakage protection and allowing various sections of the article to assume independent functions.

In order for the tapeless absorbent articles to fully exhibit their advantageous characteristics, they need to snugly fit to a user's body, particularly at leg hole portions thereof. To this end, such tapeless absorbent articles have elastically stretchable ruffles or leg gathers, as they are generally called, along peripheries of the leg holes.

One example of such tapeless absorbent articles with leg gathers is disclosed in Japanese Kokai Patent No. Hei 3-195558. Two sets of elastic members are attached along the peripheries of the leg holes to form leg gathers. Each set of elastic members extends along one leg hole from a front end of the leg hole to a midpoint of the leg hole, and continuously extends transversely of a central region (crotch region) of the article to a midpoint of the other leg to form a cross-over portion. From the midpoint of the other leg hole, each set of elastic members further extends therealong to a rear end of the other leg hole. Those two sets of elastic members are arranged so as to define a somewhat X-shaped configuration.

Such arrangement of the elastic members advantageously facilitates a continuous manufacturing process of the absorbent articles. Specifically, a liquid impermeable sheet in a continuous web form is continuously transported in one direction. Continuous elastic strands are fed onto the sheet in a sinusoidal configuration for securement thereto. The resultant combination is cut at a predetermined interval to form a composite backsheet incorporating the elastic members secured thereto.

The elastic members arranged along peripheries of the leg holes in the aforementioned, conventional tapeless absorbent article function to form leg gathers therealong. However, the elastic members extending transversely of the crotch region not only are non-functional waste material but cause the crotch region of the absorbent article to elastically stretch and contract, thereby excessively pressing against a wearer's body. Such elastic action impairs flexibility and softness in the crotch region which are the properties desired in providing comfort to the wearer during use.

One drawback of conventional tapeless absorbent articles is urine leakage from sides of the articles. Another major drawback thereof is a poor isolation or containment of fecal material. Particularly, in the above-discussed tapeless absorbent article incorporating the two sets of elastic members which traverse the crotch region thereof, those elastic members urge the crotch region into excessively tight contact with the wearer's body. Such article design has a poor fecal material containment, so that the fecal material spreads over the article.

It is an object of the present invention to provide an improved absorbent article which is capable of eliminating the above-described disadvantages that conventional articles possess, so that any undesirable deformation of the crotch region due to the contracting force of the elastic members can be avoided, and the effective containment of fecal material in the crotch region is insured.

This object is achieved by the features in claim 1.

In accordance with the present invention, there is provided an absorbent article which is provided with a main body having a waist hole and a pair of leg holes. The main body comprises a liquid impermeable backsheet, a liquid permeable topsheet and an absorbent core interposed between the backsheet and the topsheet. The absorbent article is further provided with a waist gather disposed along the waist hole, and a leg gather disposed along each of the pair of leg holes.

The topsheet includes two layers of sheet material, and two sets of elastic members interposed between the two sheets of material to form a composite topsheet. The topsheet further includes communicating means formed so as to penetrate through said sheet material.

Each of the two sets of elastic members extend along a periphery of one of the leg holes from one end thereof to a midpoint thereof, and continuously extend therefrom along a periphery of the communicating means to a midpoint of the other leg hole. From the midpoint, each set of elastic members extend to one end of the other leg hole, so that two sets of elastic members are arranged to define an X-shaped configuration.

In accordance with the absorbent article of the present invention, the topsheet has communicating means located in the crotch region of the absorbent article. The elastic members are disposed to extend along the periphery of the communicating means to form the leg gathers. The communicating means serves as an inlet for a pocket defined between the topsheet and the backsheet for receiving urine and fecal material. This provides comfort to a wearer during use. Furthermore, the arrangement of a part of all of the elastic members which form the leg gathers along the periphery of the communicating means can help provide shape and positional stability of the pocket communicating means, which provides further comfort to the wearer during use.

The elastic members which form the leg gathers are disposed to extend primarily around the leg holes, and are not located in the crotch region where elastic members are not required to be present. Accordingly, the absorbent article has softness and flexibility, and provides a snug fit and comfort to the wearer during use, with its leg gathers effectively preventing leakage from the article. Furthermore, as the elastic members are not bonded directly to the backsheet, they are not viewed or observed from outside the article. This helps provide the article with a good appearance in its crotch region.

One embodiment of the present invention will be explained hereinafter with reference to the drawings.

### BRIEF DESCRIPTION OF THE DRAWINGS

FIGURE 1 is a perspective view illustrating one embodiment of an absorbent article in accordance with the present invention.

FIGURE 2 is a developed plan view of the absorbent article of FIGURE 1.

FIGURE 3 is a cross-sectional view taken along a line A-A of Figure 2.

FIGURE 4 is a cross-sectional view taken along a line B-B of Figure 2.

FIGURE 5 is a fragmentary enlarged, perspective view of the absorbent article of Figure 1.

FIGURE 6 is a perspective view illustrating the positional relationship of a topsheet and elastic members incorporated in the absorbent article.

FIGURE 7 is a developed, plan view illustrating a topsheet incorporated in a second embodiment of the absorbent article in accordance with the present invention.

FIGURE 8 is a developed, plan view illustrating a topsheet incorporated in a third embodiment of the absorbent article in accordance with the present invention.

FIGURE 9 is an explanatory view illustrating the positional relationship of sheet materials and elastic members during a process of manufacturing the topsheet of Figure 8.

FIGURE 10 is a developed, plan view illustrating a topsheet incorporated in a fourth embodiment of the absorbent article in accordance with the present invention.

FIGURE 11 is a developed, plan view illustrating a topsheet incorporated in a fifth embodiment of the absorbent article in accordance with the present invention.

FIGURE 12 is a developed, plan view illustrating a topsheet incorporated in a sixth embodiment of the absorbent article in accordance with the present invention.

FIGURE 13 is a developed, plan view illustrating a topsheet incorporated in a seventh embodiment of the absorbent article in accordance with the present invention.

FIGURE 14 is an enlarged, cross-sectional view taken along a line C-C of Figure 13.

FIGURE 15 is a developed, plan view illustrating a topsheet incorporated in a eighth embodiment of the absorbent article in accordance with the present invention.

FIGURE 16 is a cross-sectional view taken along a line D-D of Figure 15.

FIGURE 17 is a developed, plan view illustrating a topsheet incorporated in a ninth embodiment of the absorbent article in accordance with the present invention.

FIGURE 18 is a cross-sectional view taken along a line E-E of Figure 17.

FIGURE 19 is a cross-sectional view taken along a line F-F of Figure 17.

FIGURES 20 to 23 illustrate other absorbent articles embodying the present invention in cross section to reveal the structure of a portion of the leg gather at one side of an article.

### DESCRIPTION OF THE PREFERRED EMBODIMENT

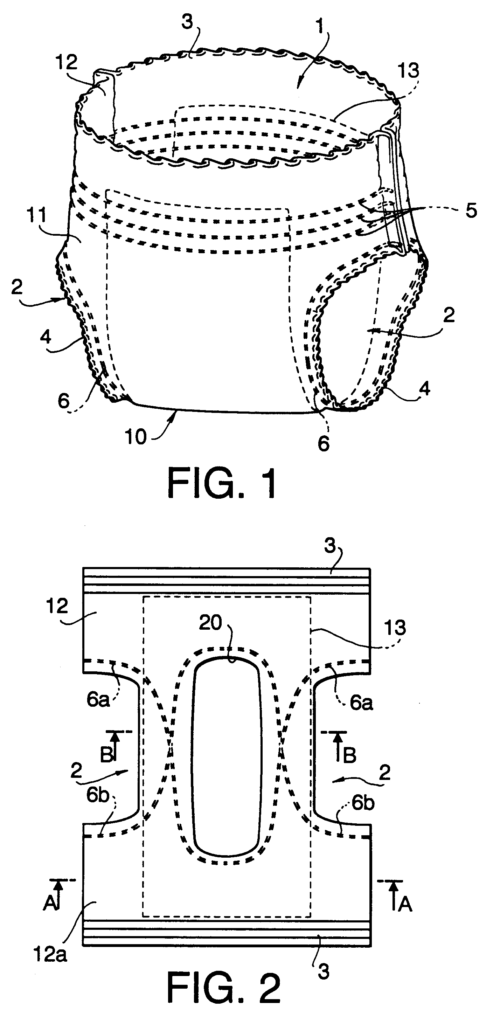

Figure 1 is a perspective view illustrating one embodiment of a tapeless absorbent article in accordance with the present invention. Figure 2 is a plan view illustrating the absorbent article of Figure 1 prior to folding and sealing the respective side panels thereof to each other.

The absorbent article as illustrated in Figure 1 comprises a main body 10 of a pant-type which has a waist hole 1 and a pair of leg holes 2. The main body 10 includes a waist gather 3 disposed along the periphery of the waist hole 1, and a leg gather 4 disposed along the periphery of each of the leg holes 2. The leg gather 4 is formed by the contracting force of a number of elastic members 6 incorporated therein. A reference numeral 5 indicates elastic members optionally provided for improved fitting of the main body to a waist portion of a wearer.

The main body 10 comprises a backsheet 11 formed of liquid impermeable material, a topsheet 12 formed of liquid impermeable material, preferably hydrophobic material, and an absorbent core 13 interposed therebetween.

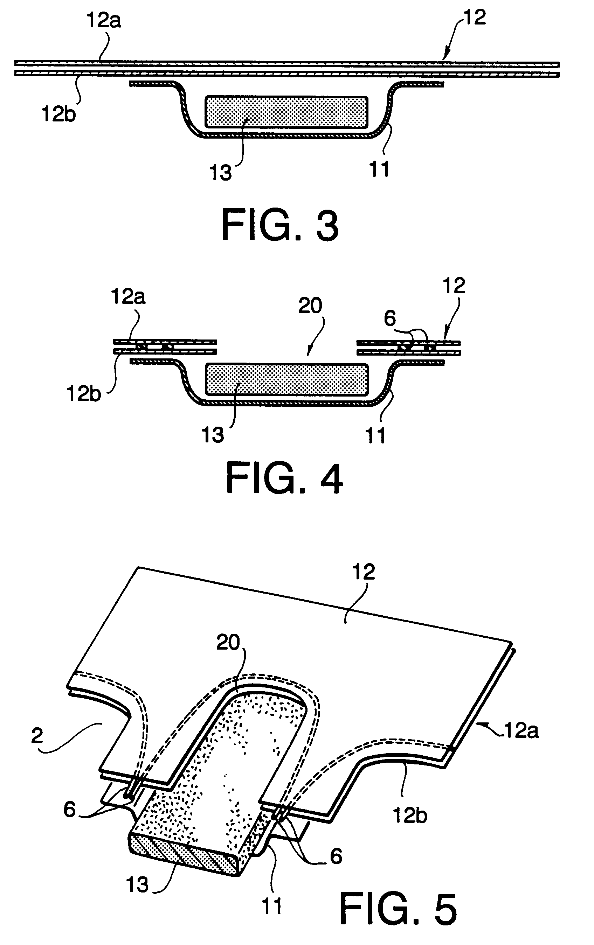

As illustrated in Figures 3 through 5, the topsheet 12 comprises a first layer 12a positioned in facing relation to a wearer's body, and a second layer 12b positioned in facing relation to the backsheet 11. A communicating means, such as a through aperture 20 is disposed in a crotch area of the garment area located between the pair of leg holes 2.

In the illustrated embodiment, the elastic members 6 extend inwardly of and along right and left contoured edges of the absorbent article to form the pair of right and left leg gathers 4, and comprise separate, first and second sets of elastic members 6a, 6b. The first set of elastic members 6a meets or crosses the second set of elastic members 6b at a midpoint of one of the leg holes 2, and continuously extends therefrom along a half peripheral portion of the aperture 20 to a midpoint of the other of the leg holes 2. From the midpoint, the first set of elastic members 6a further extends to one end of the other of the leg holes 2. The second set of elastic members 6b is disposed to extend like the first sets of elastic members 6a, but with its orientation being reversed therefrom. The first and second sets of elastic members 6a, 6b are bonded in sandwiched relationship between the first and second layers 12 a, 12b.

The composite sheet of such construction can be readily manufactured by the following process.

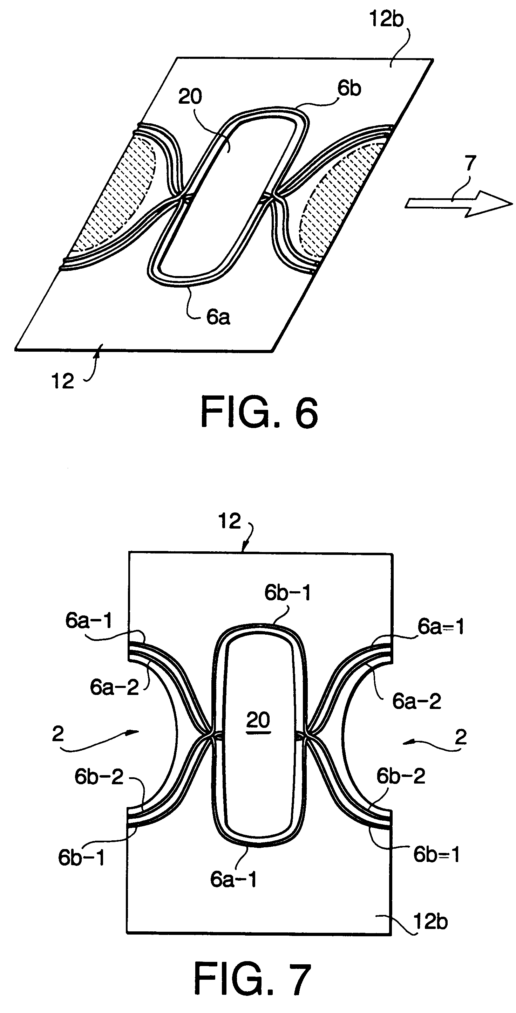

Elongated sheet material in web form that forms the second layer 12b is continuously transported in one direction, as illustrated by arrow 7 in Figure 6. The two sets of elastic members 6a, 6b are placed onto the sheet material 12b in overlapping sinusoidal configurations with an inverted phase relation to each other. A second sheet of material 12a (not shown in Figure 6) is further overlaid onto the sheet material 12b so as to sandwich the elastic members therebetween. The second sheet of material 12a and the elastic members are bonded to the sheet material 12b by conventional bonding means to form a composite sheet. The through aperture 20 is simultaneously formed which penetrates the dual-layered sheet material. The resultant composite topsheet is then cut into an individual, article unit length to which is further cut to form leg holes (shown by cross hatch lines).

The composite sheet thus formed is subsequently combined with the backsheet 11, the absorbent core 13, and the other article elements to manufacture the absorbent articles in accordance with conventional manufacturing processes. The relative positions of the layered materials within the garment are illustrated in the cross-sectional views Figs. 3-5.

The elastic members 6 generally comprise a number of parallel rubber threads, or strands, to form a leg gather which is flexible and has sufficient elastic contractibility to provide leakage protection. In a particular embodiment, some of the rubber threads can be disposed to traverse the aperture 20 while the remaining rubber threads are disposed to extend along the periphery of the aperture 20.

In the embodiment as shown in Figure 7, the first and second sets of elastic members 6a, 6b are disposed to extend along peripheries of the leg holes 2, and respectively comprise first, two rubber threads 6ₐ₋₁, 6_{b-2} and second, two rubber threads 6_{b-1}, 6_{b-2}, as similar to the embodiment of Figure 6. The rubber threads 6ₐ₋₁, 6_{b-1,} which are positioned more remote from the peripheries of the leg holes 2 extend between the pair of leg holes 2 along the periphery of the aperture 20. However, the rubber threads 6ₐ₋₂, 6_{b-2} which are positioned closer to the peripheries of the leg holes 2 are discontinued at the periphery of the aperture 20 by cutting.

In such construction, the contracting force of the elastic members 6 along the periphery of the aperture 20 is controlled to be smaller than that along the peripheries of the leg holes 2, so that the inlet of the pocket provided by the aperture 20 may not be excessively restricted or closed. The contracting forces along the periphery of the aperture 20 and along the peripheries of the leg holes 2 can be selectively controlled by setting a ratio of the number of rubber threads which form the elastic members 6 to the number of rubber threads which are severed adjacent the periphery of the aperture 20 within a proper range.

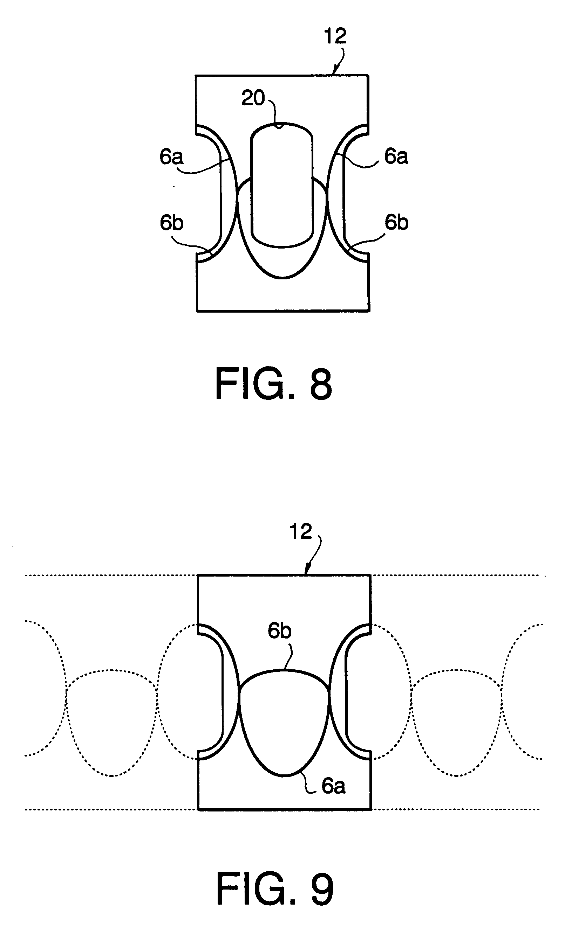

Figure 8 illustrates the topsheet 12 in still another embodiment of the absorbent article in accordance with the present invention. In this embodiment, the first set of elastic members 6a extends along the periphery of the aperture 20 while the second set of elastic members 6b is discontinued at the periphery of the aperture 20 by cutting.

The topsheet 12 of such construction can be readily manufactured by a continuous process. Typically, during the process that the two sets of elastic members 6a, 6b are positioned between the two sheet materials in continuous web forms for attachment thereto, the amplitude of the sinusoidal configuration that the first set of elastic members 6a describes is set greater, and the amplitude of the sinusoidal configuration that the second set of elastic members 6b describes is set smaller, as illustrated in Figure 9. The aperture 20 is formed in the sheet material by cutting which simultaneously splits the second set of elastic members 6b at a periphery of the aperture as shown.

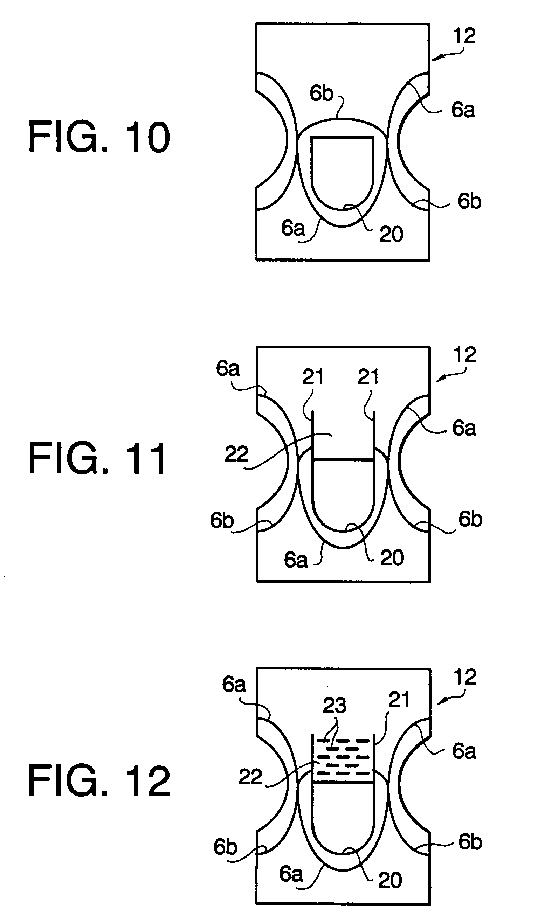

Figure 10 illustrates a fourth embodiment of the absorbent article wherein the aperture 20 is formed within an area surrounded by the two sets of elastic members 6a, 6b in a crotch region of the topsheet 12 as illustrated in Figure 9.

Figure 11 illustrates a fifth embodiment of the absorbent article which incorporates slits 21 extending from respective sides of the aperture 20 to form a flap 22 in the topsheet 12 Figure 10. A number of slits 23 may be additionally placed in the flap 22 in accordance with a sixth embodiment of the absorbent article as illustrated in Figure 12.

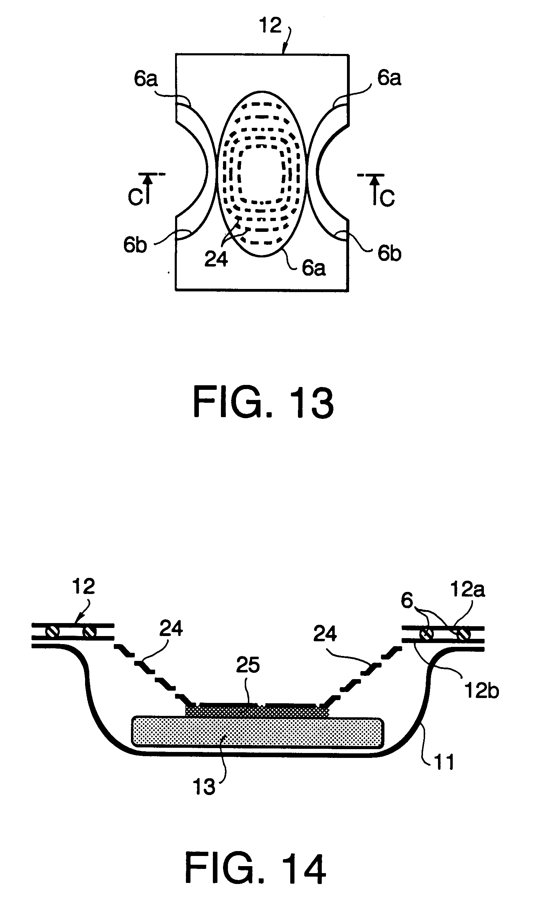

Figures 13 and 14 illustrate the topsheet 12 incorporated in a seventh embodiment of the absorbent article. In this embodiment, the two sets of elastic members 6a, 6b define a substantially oblong crotch area, and a number of slits 24 are formed within and along a periphery of the area. Those slits 24 permit the elevation of a central area surrounded thereby in a thickness direction of the topsheet 12 as the slits 24 open up. The central area is bonded to the absorbent core 13 by appropriate bonding means such as hot-melt-type adhesives 25.

Accordingly, the topsheet 12 defines a recess in the crotch region of the article which serves as a pocket for receiving and containing body wastes.

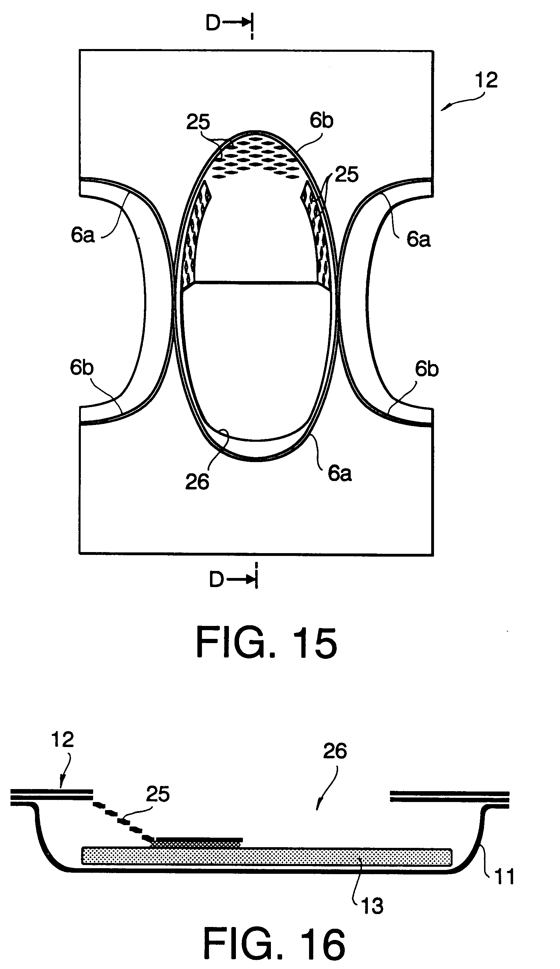

Figure 15 and 16 illustrate an eighth embodiment of the absorbent article wherein about a half part of the substantially oblong crotch region as shown in Figure 13 is cut out to form an aperture 26. The aperture 26 is positioned in a rear section of the absorbent article in use so as to serve as a pocket of high containment capacity for receiving and containing body wastes, particularly fecal material.

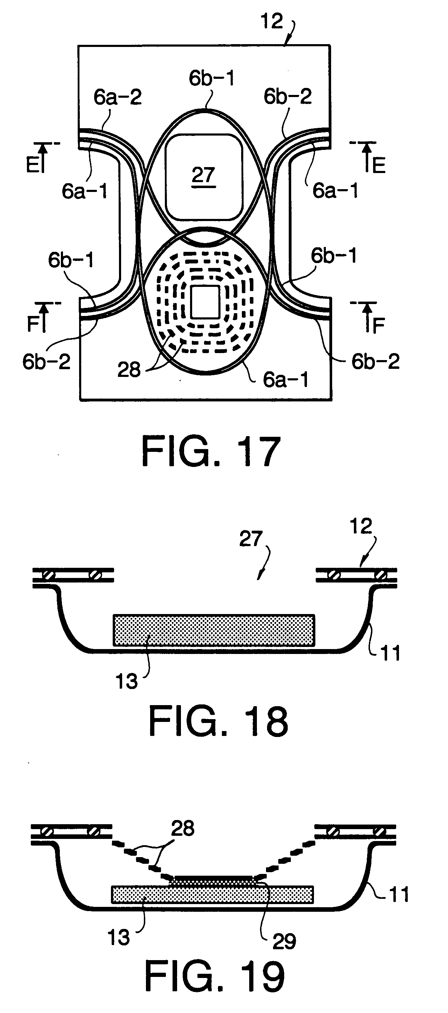

Figures 17 through 19 illustrate a ninth embodiment of the absorbent article in accordance with the present invention. The first and second sets of elastic members 6a, 6b respectively comprise first, two rubber threads 6ₐ₋₁, 6ₐ₋₂, and second, two rubber threads 6_{b-1}, 6_{b-2}. The rubber threads 6ₐ₋₁, 6_{b-1} are disposed to extend outwardly of and along the substantially oblong, crotch region, while the rubber threads 6ₐ₋₂, 6_{b-2} are disposed to extend transversely of a central portion of the crotch region. Accordingly, the crotch region is divided into a pair of first and second regions which are respectively surrounded by rubber threads. The first region includes an aperture 27, and the second region includes a number of slits 28 arranged in a number of circles. A topsheet portion surrounded by the slits 28 is bonded onto the absorbent core 13 by hot-melt-type adhesives 29, so that a recess is defined in the absorbent article.

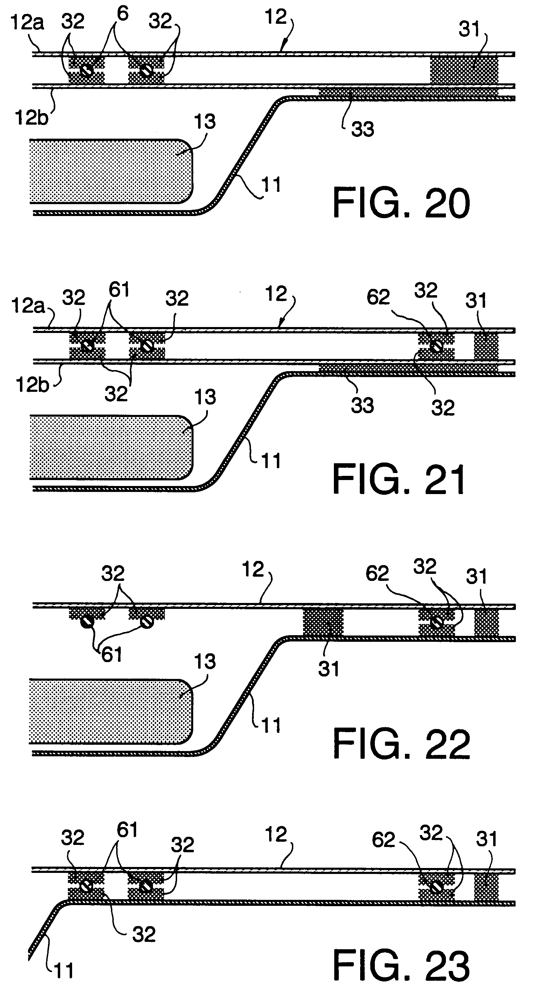

The absorbent article shown in Figures 20 comprising a pair of sheets of material 12a and 12b joined together means of a layer 31 of a hot-melt-type adhesive, and an absorbent core 13. Elastic members 6 are sandwiched between the members 12a and 12b of the topsheet 12 and bonded thereto by hot-melt-type adhesive layers 32. Numeral 33 depicts a layer of hot-melt-type adhesive provided for bonding the backsheet and topsheet. In this embodiment, the elastic members 6 are not connected to the backsheet 11.

The foregoing description has related to an absorbent article in which top sheet has a dual-layered construction. This has an advantage in production because the elastic members may easily be held between the two layers. However, the present invention allows the use of a topsheet of single layer material, if the elastic members can be stably secured on the topsheet. Explaining further, because the tension in the portions of the elastic members surrounding the central apertured hole may be less than that surrounding the leg holes, it is possible to secure them to the topsheet by an adhesive, while the portions surrounding the leg holes are supported by the backsheet.

In the embodiment shown in Figure 21, inner elastic members 61 and outer elastic member 62 are disposed between a pair of materials 12a and 12b constituting a topsheet 12, the materials 12a and 12b being joined together by means of a layer of an adhesive 31. The outer elastic member 62 is connected to the backsheet 11 by adhesive 32, while the inner elastic members 61 are not connected to the backsheet. A portion of the topsheet 12 is secured to the backsheet 11 by a layer 33 of a hot-melt-type adhesive.

An absorbent article shown in Figure 22 is different from that shown in Figure 21 in that the topsheet 12 comprises a single member secured to backsheet 11 by adhesive 31.

Alternatively, as shown in Figure 23, all the elastic members 61 and 62 may be arranged between the backsheet 11 and topsheet 12 and connected thereto by adhesive layers 32.

As described above, the absorbent article of the present invention comprises a topsheet which includes communicating means in the crotch region of the article, and elastic members for forming the leg gathers are disposed to extend along the periphery of the communicating means. The communicating means serves as the inlet of the pocket defined between the topsheet and the backsheet for receiving urine and fecal material. This provides comfort to the wearer during use. Since the elastic members for forming the leg gathers are disposed to extend along the periphery of the communicating means, the improved positional and shape stabilities of the communicating means are obtained.

The elastic members which form the leg gathers are disposed to extend primarily around the leg holes, and are not located in the crotch region where the elastic members are not required to be present. Accordingly, the absorbent article has softness and flexibility, and provides a snug fit and comfort to the wearer during use, with its leg gathers effectively preventing leakage from the article. Furthermore, as the elastic members are not bonded to the backsheet, the backsheet is not gathered or corrugated by the elastic members. This helps provide the article with a good appearance in its crotch region of the absorbent article.

## Claims

1. An absorbent article comprising:
a main body (10) constructed to provide a waist hole (1) and a pair of leg holes (2), and a crotch region, said main body comprising a topsheet (12) for facing toward a wearer's body, a backsheet (11) disposed outwardly of the topsheet, and an absorbent core (13) interposed between the topsheet and the backsheet,
said topsheet (12) having elastic members (6a, 6b) secured thereto, and communicating means (20) having a periphery for permitting fecal material to pass therethrough in the crotch area of the article,
characterized by a first set of elastic members (6a) extending along a periphery of a first leg hole (2) from one end thereof to a midpoint thereof, continuously extending therefrom along one portion of said periphery of the communicating means to a midpoint of a second leg hole (1), and further extending therefrom to one end of the second leg hole (1) so that said first set of elastic members (6a) are disposed in a substantially U-shaped configuration, and
a second set of elastic members (6b) being disposed to extend along peripheral portions of said first and second leg holes (2) where said first set of elastic members (6a, 6b) are not disposed, wherein
a leg gather (4) is disposed along each of the leg holes.

2. An absorbent article according to claim 1, wherein a waist gather (3) is disposed along the waist hole (1); and said topsheet (12) comprises a dual-layered sheet material (12a, 12b), the first and second sets of elastic members (6a, 6b) being interposed between the dual sheet material.

3. The absorbent article of claim 1 or 2, wherein
said second set of elastic members (6b) is disposed to extend along the other portion of the periphery of said communicating means (20) to define an inverted U-shaped configuration.

4. The absorbent article of claims 1 or 2, wherein
said communicating means (20) comprises a through aperture formed in said sheet materials (12a, 12b) or topsheet for penetrating therethrough.

5. The absorbent article of claim 4, wherein
said through aperture (20) has openings of different sizes in front and rear sides of the article.

6. The absorbent article of claims 1 or 2, wherein
said communicating means comprises a number of slits (23, 24, 25 or 28) disposed in a substantially circular area adjacent edges of said crotch region for penetrating said topsheet (12) or sheet materials (12a, 12b).

7. The absorbent article of claims 1 or 2, wherein
said communicating means comprises first and second portions, said first portion comprising an aperture formed in a front portion of the article penetrating the topsheet or sheet materials (12a, 12b), said second portion comprising a number of slits (23, 24, 25 or 28) formed in a rear portion of the article penetrating the topsheet (12) or sheet materials.

8. The absorbent article according to a preceeding claim, wherein said topsheet (12) is bonded to said absorbent core at the area surrounded by said slits (23, 24, 25 or 28).

9. The absorbent article of claim 7 or 8, wherein
said elastic members (6a, 6b) comprise a number of elastic strands, a portion of the elastic strands extending along the edges of said crotch region, and remaining portions thereof extending between the first and second portions of said communicating means (20).

## Patentansprüche

1. Absorbierendes Produkt, umfassend einen Hauptkörper (10), der eine Leiböffnung (1) und ein Paar von Beinöffnungen (2) sowie einen Gabelungsbereich aufweist, wobei der Hauptkörper eine obere Lage (12), die dem Körper des Trägers zugewandt ist, eine äußere Lage (11), die nach außen von der oberen Lage angeordnet ist, und einen absorbierenden Kern (13) aufweist, der zwischen der oberen Lage und der äußeren Lage angeordnet ist, wobei ferner die obere Lage (12) daran angebrachte elastische Elemente (6a, 6b) und eine Verbindungseinrichtung (20) aufweist, die einen Umfang hat, damit Fäkalmaterial in den Gabelungsbereich des Produkts hindurchgelangen kann,
gekennzeichnet durch
einen ersten Satz von elastischen Elementen (6a), die sich längs des Umfangs der ersten Beinöffnung (2) von deren einem Ende bis zu einer mittleren Stelle von dieser und weiter längs eines Abschnitts des Umfangs der Verbindungseinrichtung zu einer mittleren Stelle der zweiten Beinöffnung (1) erstreckt und weiter zum einen Ende der zweiten Beinöffnung (1), so daß dieser erste Satz von elastischen Elementen (6a) in einer im wesentlichen U-förmigen Gestalt angeordnet ist, und
einen zweiten Satz von elastischen Elementen (6b), die sich längs jener Umfangsabschnitte der ersten und zweiten Beinöffnungen (2) erstrecken, an denen der erste Satz von elastischen Elementen (6a) nicht vorgesehen ist, wobei eine Raffung (4) längs jeder Beinöffnung vorgesehen ist.

2. Absorbierendes Produkt nach Anspruch 1, wobei eine Raffung (3) längs der Leiböffnung (1) vorgesehen ist und die obere Lage (12) ein zweilagiges Bahnmaterial (12a, 12b) umfaßt, wobei die ersten und zweiten Sätze von elastischen Elementen (6a, 6b) zwischen dem doppellagigen Bahnmaterial angeordnet sind.

3. Absorbierendes Produkt nach Anspruch 1 oder 2, wobei der zweite Satz von elastischen Elementen (6b) längs des anderen Abschnitts des Umfangs der Verbindungseinrichtung (20) angeordnet ist, um eine umgekehrte U-förmige Gestalt zu bilden.

4. Absorbierendes Produkt nach Anspruch 1 oder 2, wobei die Verbindungseinrichtung (20) eine durchgehende Öffnung aufweist, die in den Bahnmaterialien (12a, 12b) oder in der oberen Lage zum Durchdringen von dieser ausgebildet ist.

5. Absorbierendes Produkt nach Anspruch 4, wobei die durchgehende Öffnung (20) Öffnungen von unterschiedlicher Größe auf der Vorder- und Rückseite des Produkts aufweist.

6. Absorbierendes Produkt nach Anspruch 1 oder 2, wobei die Verbindungseinrichtung eine Anzahl von Schlitzen (23, 24, 25 oder 28) aufweist, die in einem im wesentlichen kreisförmigen Bereich angrenzend an die Ränder des Gabelungsbereichs angeordnet sind, um die obere Lage (12) oder die Bahnmaterialien (12a, 12b) zu durchdringen.

7. Absorbierendes Produkt nach Anspruch 1 oder 2, wobei die Verbindungseinrichtung erste und zweite Abschnitte umfaßt und der erste Abschnitt eine Öffnung aufweist, die in einem vorderen Abschnitt des Produkts ausgebildet ist und die obere Lage oder die Bahnmaterialien (12a, 12b) durchdringt, während der zweite Abschnitt eine Anzahl von Schlitzen (23, 24, 25 oder 28) aufweist, die in einem hinteren Teil des Produkts ausgebildet sind, um die obere Lage (12) oder die Bahnmaterialien zu durchdringen.

8. Absorbierendes Produkt nach einem vorhergehenden Anspruch, wobei die obere Lage (12) an den absorbierenden Kern in einem Bereich gebunden ist, der durch die Schlitze (23, 24, 25 oder 28) umgeben ist.

9. Absorbierendes Produkt nach Anspruch 7 oder 8, wobei die elastischen Elemente (6a, 6b) eine Anzahl von elastischen Bänder umfassen, wobei sich ein Abschnitt der elastischen Bänder längs der Ränder des Gabelungsbereichs erstreckt und die verbleibenden Abschnitte von diesen sich zwischen den ersten und zweiten Abschnitten der Verbindungseinrichtung (20) erstrecken.

## Revendications

1. Article absorbant comprenant :
un corps principal (10) construit pour ménager un ouverture de taille (1) et une paire d'ouvertures de jambes (2), et une région d'entrejambe, ledit corps principal comprenant une feuille supérieure (12) destinée à faire face au corps du porteur, une feuille arrière (11) disposée à l'extérieur de la feuille supérieure, et une âme absorbante (13) interposée entre la feuille supérieure et la feuille arrière,
ladite feuille supérieure (12) ayant des éléments élastiques (6a, 6b) qui y sont fixés, et des moyens de communication (20) ayant une périphérie pour permettre aux matières fécales de la traverser dans la région d'entrejambe de l'article ; caractérisé par
un premier jeu d'éléments élastiques (6a) s'étendant le long d'une périphérie de la première ouverture de jambe (2), d'une extrémité de cette périphérie jusqu'à un point milieu de cette périphérie, en s'étendant sans interruption depuis ce point le long d'une portion de ladite périphérie des moyens de communication jusqu'à un point milieu d'une deuxième ouverture de jambe (1), et se prolongeant de ce point jusqu'à une extrémité de la deuxième ouverture de jambe (1), de sorte que ledit premier jeu d'éléments élastiques (6a) sont disposés dans une configuration sensiblement en forme de U, et
un deuxième jeu d'éléments élastiques (6b) étant disposé pour s'étendre le long de portions périphériques desdites première et deuxième ouvertures de jambes (2), dans une zone dans laquelle ledit premier jeu d'éléments élastiques (6a, 6b) ne sont pas disposés, dans lequel une fronce de jambe (4) est disposée le long de chacune des ouvertures de jambes.

2. Article absorbant selon la revendication 1, dans lequel une fronce de taille (3) est disposée le long de l'ouverture de taille (1) ; et ladite feuille supérieure (12) comprend une matière en feuille à deux couches (12a, 12b), les premier et deuxième jeux d'éléments élastiques (6a, 6b) étant interposés entre les deux couches de la matière à double couche.

3. Article absorbant selon la revendication 1 ou 2, dans lequel ledit deuxième jeu d'éléments élastiques (6b) est disposé pour s'étendre le long de l'autre portion de la périphérie desdits moyens de communication (20) pour définir une configuration en forme de U inversé.

4. Article absorbant selon la revendication 1 ou 2, dans lequel lesdits moyens de communication (20) comprennent une ouverture traversante formée dans les matières en feuille (12a, 12b) ou dans la feuille supérieure pour les ou la traverser.

5. Article absorbant selon la revendication 4, dans lequel
ladite ouverture traversante (20) possède des ouvertures de différentes dimensions dans les côtés avant et arrière de l'article.

6. Article absorbant selon la revendication 1 ou 2, dans lequel
lesdits moyens de communication comprennent plusieurs fentes (23, 24, 25 ou 28) disposées dans une zone sensiblement circulaire adjacente aux bords de ladite région d'entrejambe pour traverser ladite feuille supérieure (12) ou les matières en feuille (12a, 12b).

7. Article absorbant selon la revendication 1 ou 2, dans lequel
lesdits moyens de communication comprennent des première et deuxième portions, ladite première portion comprenant une ouverture formée dans une portion avant de l'article, en traversant la feuille supérieure ou les matières en feuille (12a, 12b) et ladite deuxième portion comprenant plusieurs fentes (23, 24, 25 ou 28) formées dans une portion arrière de l'article en traversant la feuille supérieure (12) ou les matières en feuille.

8. Article absorbant selon une revendication précédente, dans lequel
ladite feuille supérieure (12) est fixée à ladite âme absorbante dans la zone entourée par lesdites fentes (23, 24, 25 ou 28).

9. Article absorbant selon la revendication 7 ou 8, dans lequel
lesdits éléments élastiques (6a, 6b) comprennent plusieurs cordons élastiques, une portion des cordons élastiques s'étendant le long des bords de ladite région d'entrejambe, et des portions restantes de ces cordons s'étendant entre les première et deuxième portions desdits moyens de communication (20).
